# EUROPÄISCHE PATENTSCHRIFT

(11) **EP 3 044 305 B1**
(45) Veröffentlichungstag und Bekanntmachung des Hinweises auf die Patenterteilung: **15.03.2017**
(21) Anmeldenummer: 14758819.8
(22) Anmeldetag: 21.08.2014
(51) Int. Cl.: C12M 1/00, C12M 1/21, C12M 1/34

(54) **BEHÄLTER MIT FLEXIBLER WANDUNG**
CONTAINER WITH FLEXIBLE WALLS
CONTENEUR À PAROI FLEXIBLE

(30) Priorität: 09.09.2013 DE 102013109820
(43) Veröffentlichungstag der Anmeldung: 20.07.2016
(73) Patentinhaber: Sartorius Stedim Biotech GmbH, 37079 Göttingen (DE)
(72) Erfinder: KEITEL, Heinz-Rüdiger, verstorben (DE); HUSEMANN, Bernward, 37079 Göttingen (DE); KAHLERT, Wolfgang, 34327 Körle (DE)
(74) Vertreter: Schneider, Peter Christian
(86) Internationale Anmeldenummer: PCT/EP2014/067856
(87) Internationale Veröffentlichungsnummer: WO 2015/032629

(56) Entgegenhaltungen:
- WO-A1-2009/093995
- WO-A1-2011/082787
- WO-A1-2011/112680
- DE-A1-102006 018 824
- DE-A1-102007 036 049
- DE-A1-102009 037 345
- DE-A1-102010 007 559
- US-A1- 2013 101 982

## Beschreibung

### Gebiet der Erfindung

Die Erfindung betrifft einen Behälter mit flexibler Wandung und mindestens einem in den von der flexiblen Wandung umgebenen Behälterinnenraum hineinragenden elektrischen Sensor, bei dem zwischen mindestens zwei elektrisch leitenden Plättchen die Leitfähigkeit oder Impedanz eines die Plättchen umgebenden Mediums ermittelbar ist, wobei die Plättchen über Verbindungsleitungen mit einer außerhalb des Behälterinnenraumes angeordneten Steuer- und Regeleinheit verbunden sind.

### Stand der Technik

Aus der US 2011/0187388 A1 ist ein Bioreaktor mit einem Behälter mit flexibler Wandung und mindestens einem um den von der flexiblen Wandung umgebenden Behälterinnenraum hineinragenden elektrischen Sensor bekannt. Über elektrisch leitende Plättchen oder Elektroden kann dabei die Leitfähigkeit oder Impedanz eines die Plättchen umgebenden Mediums ermittelt werden, wobei die Plättchen über Verbindungsleitungen mit einer außerhalb des Behälterinnenraumes angeordneten Steuer- und Regelungseinheit verbunden sind.

Nachteilig dabei ist, dass die Sensoren bzw. ihre Elektrodenflächen unmittelbar an der Wandung angeordnet sind. Dies ermöglicht zwar, ein Zusammenfalten des Behälters mit den Sensoren, hat aber den Nachteil, dass der oder die Sensoren nicht an unterschiedliche Füllstände angepasst werden können.

Weiterhin ist aus der US 2011/0187388 A1 ein Behälter mit einer starren Wandung bekannt, in dessen Behälterinnenraum ein Sensor am freien Ende einer starren zylindrischen Umhüllung angeordnet ist, die in einem eine Deckelwandung durchdringenden Adapter verschieblich gelagert ist.

Nachteilig dabei ist, dass zwischen der verschieblichen Hülle und dem Adapter Undichtigkeiten und Kontaminationen entstehen können. Insbesondere kommt es bei einer Verschiebung der Umhüllung von außen in den Innenraum hinein zu einer Kontamination. Soweit die zylindrische Umhüllung mit dem Adapter nicht verschieblich verbunden ist, kann die Höhe des Sensors nicht verschoben werden, so dass eine Anpassung an unterschiedliche Füllstände nicht möglich ist. Zudem ist es nicht möglich, einen Bioreaktor mit einem Behälter mit flexibler Wandung und einem in einer starren zylindrischen Umhüllung angeordneten Sensor, der in seiner Position bezüglich des Füllstandes fixiert ist, flach zusammenzufalten.

Aus der DE 41 42 967 A1 ist ein Bioreaktor bekannt, dessen Behälter eine starre Wandung und einen Deckel aufweist, durch den zur Bekämpfung von Schaum von außen eine zylindrische Messsonde bis zur maximalen Füllhöhe des Bioreaktors geführt ist. Mit Hilfe dieser Sonde werden der sich auf der Oberfläche der Fermenterbrühe bildende Schaum und dessen Höhe erfasst. Das Messsignal der Sonde wird einer Regelvorrichtung zugeleitet, die außerhalb des Behälters angeordnet ist. In Abhängigkeit von der Höhe des Schaumes über der Fermenterbrühe, wird eine in eine Zuleitung eingebaute Pumpe betätigt, mit der aus einem Behälter Antischaummittel dem Bioreaktor zugeführt wird.

Nachteilig auch bei diesem bekannten Bioreaktor ist, dass bei einer verschieblichen Sonde Probleme mit der Abdichtung und Kontamination zwischen Sonde und Deckel bzw. Behälterinnenraum auftreten. Soweit die Sonde mit dem Deckel fest verbunden ist, ist es nicht mehr möglich, die Füllhöhe zu verstellen. Soweit mit einer solchen Sonde unterschiedliche Füllhöhen bei fixer Sondenanordnung abgedeckt werden sollen, ist die Sonde relativ kompliziert und kostenintensiv aufgebaut.

Aus der US 5,922,112 A ist ein oben offener Bioreaktor für Abwasser bekannt, der einen von oben in den Behälterinnenraum hineinragenden elektrischen Sensor zur Messung von Schaum aufweist. Der Sensor ist über eine Steuer- und Regeleinheit mit einer Pumpe verbunden, die in einer Leitung zwischen einem Behälter mit einem Antischaummittel und dem Bioreaktor angeordnet ist.

Weiterhin ist aus der DE 10 2010 007 559 A1 ein Bioreaktor mit einem Behälter mit flexibler Wandung bekannt, der zur Schaummessung einen seitlich von außen anordenbaren optischen Sensor aufweist. Dieser optische Sensor, der sich grundsätzlich bewährt hat, ist wiederverwendbar, da er mit dem Behälterinnenraum und der darin befindlichen Flüssigkeit und einer evtl. Schaumschicht nicht in Berührung kommt und weil er durch ein transparentes Fenster, das in der Behälterwandung angeordnet ist, getrennt vom Behälterinnenraum ist. Ein solcher optischer Sensor ist, insbesondere wenn er unterschiedliche Füllstände erkennen kann, relativ kostenintensiv.

Aus der WO 2011/112680 A1 ist ein Behälter mit flexibler Wandung und mindestens einem in den von der flexiblen Wandung umgebenen Behälterinnenraum hineinragenden Sensor, beispielsweise zur Messung der Leitfähigkeit eines im Behälter befindlichen Mediums, bekannt. Der Sensor ist über eine elektrische Verbindungsleitung mit einem außerhalb des Behälterinnenraumes angeordneten Messgerät verbunden.

Nachteilig bei dem bekannten Behälter ist, dass der Sensor, vor der Benutzung in ein Sensorgehäuse eingebracht und fixiert werden muss. Dabei können zum einen Dichtprobleme auftreten und zum anderen sind weder der Sensor noch das Sensorgehäuse in ihrer Länge verstellbar, so dass auch keine Höhenverstellung des Sensors bzw. seiner Elektroden im Behälterinnenraum möglich ist.

Aus der WO 2011/082787 A1 ist ein Behälter mit flexibler Wandung und einem Wandungsdurchbruch, durch den mit Hilfe einer Entnahmevorrichtung eine Komponente aus dem Behälter entnommen werden kann, bekannt. Ein Rührwerksmast kann dabei mit seinem freien Ende aus dem Wandungsdurchbruch herausragen, wobei das freie Ende von einer - außerhalb des Behälterinnenraumes angeordneten - flexiblen Umhüllung umgeben ist, die an einem ersten Ende durch eine Verschlussvorrichtung verschlossen ist und an ihrem dem ersten Ende abgewandten zweiten Ende mit der den Wandungsdurchbruch umgebenden Wandung fluiddicht verbunden ist.

Nachteilig bei der aus der WO 2011/082787 A1 bekannten Entnahmevorrichtung ist, dass zur Entnahme der Komponente zum einen eine zusätzliche Abschnüreinrichtung im Bereich des zweiten Endes und zum anderen die Verschlussvorrichtung im Bereich des ersten Endes notwendig sind. Nachteilig ist zudem, dass, wenn auch nur geringfügig, Gas aus dem Behälterinnenraum entweichen kann.

Aus der DE 10 2006 018 824 A1 ist es bekannt, Einweg-Bioreaktoren zu verwenden, deren flexible Wandung Durchführungen aufweist, die geeignet sind, Sensoren für die Messung der Temperatur, des Gasgehaltes, der Innenkonzentrationen, der optischen Eigenschaften, der Partikelkonzentration und der Zellvitalität zum Zwecke der Prozesskontrolle mit dem Medium bzw. dem Gasraum in Kontakt zu bringen. Die Sensoren werden üblicherweise mit einer Schraubverbindung auf den Stutzen befestigt und an den Innenflanken der Durchführungen mittels eines O-Ringes abgedichtet.

Aus der WO 2009/093995A1 ist ebenfalls ein flexibler Bioreaktor bekannt, der einen an seiner Wandung angeordneten Sensor aufweist, der mit einem Kontrollsystem verbunden ist. Der Sensor kann beispielsweise als Schaumsensor ausgebildet sein. Der Bioreaktor ist faltbar und als Einwegbeutel ausgebildet.

### Aufgabenstellung

Es ist die Aufgabe der vorliegenden Erfindung, einen Bioreaktor zur Verfügung zu stellen, der einen Behälter mit flexibler Wandung und mit mindestens einem kostengünstigen elektrischen Sensor zur Ermittlung der Leitfähigkeit oder Impedanz eines im Behälter angeordneten Mediums aufweist. Dabei soll der Behälter mit Sensor zur Einwegverwendung geeignet und im leeren Zustand volumenarm zusammenfaltbar sein.

### Darlegung der Erfindung

Diese Aufgabe wird in Verbindung mit den Merkmalen des Oberbegriffs von Anspruch 1 dadurch gelöst, dass mindestens ein erstes Plättchen an einem geschlossenen, freien Ende einer ersten zylindrischen Umhüllung mit einer zum umgebenden Medium freiliegenden Kontaktfläche angeordnet ist und dass die Umhüllung in ihrer Länge verstellbar und als ein flexibler Schlauch oder Faltenbalg ausgebildet ist, durch den die elektrische Verbindungsleitung des Plättchens nach außen geführt wird. Der Sensor soll zudem leicht zu positionieren sein.

Durch die Verwendung von mindestens zwei Plättchen zwischen denen die Leitfähigkeit oder Impedanz eines die Plättchen umgebenden Mediums ermittelbar ist, wird ein einfacher, kostengünstiger Aufbau eines elektrischen Sensors ermöglicht, der damit auch zur Verwendung als Einwegsensor geeignet ist. Durch die Anordnung mindestens eines ersten Plättchens an einem geschlossenen freien Ende einer ersten zylindrischen Umhüllung lässt sich das mindestens eine Plättchen einfach an einem vorgesehenen Ort im Behälterinnenraum positionieren. Durch die Ausbildung der Umhüllung als einen flexiblen Schlauch oder Faltenbalg lässt sich ein leerer Behälter volumenarm zusammenfalten, verpacken und sterilisieren. Unter Schlauch soll im Rahmen dieser Anmeldung auch eine schlauch- oder sackförmige Folie verstanden werden. Der Querschnitt der Umhüllung kann von der Kreisform abweichen und beispielsweise auch oval oder rechteckig ausgebildet sein. Durch die Verwendung einer Umhüllung kann zum einen die notwendige Flexibilität und zum anderen eine einfache Leitungsverbindung nach außen erreicht werden. Gleichzeitig wird die notwendige Abdichtung und Kontaminationsfreiheit gegenüber der Umwelt sichergestellt.

Gemäß einer bevorzugten Ausführungsform der Erfindung ist in der als Faltenbalg ausgebildeten zylindrischen Umhüllung ein gegenüber einem Wandungsdurchbruch längs verschiebliches Rohr angeordnet, das an seinem dem Plättchen zugewandten Ende mit dem freien Ende des Faltenbalgs verbunden ist. Das längs verschiebliche Rohr ragt dabei in einer bevorzugten Ausführungsform an seinem dem Plättchen abgewandten Ende aus dem Behälterinnenraum heraus. Anstelle des Rohres kann auch eine Führungsstange verwendet werden. Durch das längs verschiebliche Rohr wird zum einen eine nachträglich Höhenverstellung des mindestens einen Sensorplättchens möglich und zum anderen kann zum volumenarmen Zusammenfalten des Behälters das weniger flexible, längsverschiebliche Rohr weitgehend aus dem Behälterinnenraum herausgezogen werden. Durch die Verwendung eines Faltenbalges wird zudem zwischen Wandungsdurchbruch und längs verschieblichen Rohr bestehendes Dichtungs- und Kontaminationsproblem sicher gelöst. Soweit das längsverschiebliche Rohr an seinem dem Plättchen abgewandten Ende aus dem Behälterinnenraum herausragt, ist es in Längsrichtung leichter zu verschieben. Grundsätzlich kann das starre Rohr auch teleskopartig ausziehbar ausgebildet sein.

Nach einer weiteren bevorzugten Ausführungsform der Erfindung weist der Wandungsdurchbruch ein Adapterstück auf, in dem das längs verschiebliche Rohr angeordnet ist und dessen dem Behälterinnenraum zugewandtes Ende mit dem Faltenbalg verbunden ist.

Gemäß einer weiteren bevorzugten Ausführungsform der Erfindung weist das freie Ende der ersten Umhüllung ein zweites Plättchen auf, das zum ersten Plättchen beabstandet angeordnet ist. Diese Anordnung stellt sicher, dass die zu einer Leitfähigkeits- oder Impedanzmessung notwendigen zwei Plättchen immer den gleichen Abstand zueinander aufweisen.

Nach einer weiteren bevorzugten Ausführungsform der Erfindung ist ein zweites Plättchen an dem geschlossenen, freien Ende einer zweiten zylindrischen Umhüllung mit einer zum umgebenden Medium freiliegenden Kontaktfläche angeordnet. Dies ist insbesondere von Vorteil, wenn die beiden Plättchen auf unterschiedlichen Höhen oder schräg oder quer zueinander angeordnet werden sollen.

Nach einer weiteren bevorzugten Ausführungsform der Erfindung ist ein zweites Plättchen direkt an der dem Behälterinnenraum zugewandten Innenseite der flexiblen Wandung angeordnet und weist eine von der Wandung abgewandte freiliegende Kontaktfläche auf. Das zweite Plättchen ist dabei an der Wandung fest fixiert und weist damit eine feste Position auf.

Gemäß einer weiteren bevorzugten Ausführungsform der Erfindung sind die Kontaktflächen der Plättchen oberhalb eines vorgesehenen Flüssigkeitspegels angeordnet. Damit lässt sich zum einen oberhalb des Flüssigkeitspegels befindlicher Schaum detektieren und zum anderen lässt sich der Anstieg des Flüssigkeitspegels bei Kontakt mit beiden Plättchen feststellen.

Entsprechend ist gemäß einer weiteren bevorzugten Ausführungsform der Erfindung der von mindestens zwei Plättchen gebildete Sensor zur Detektion von Flüssigkeit und/oder Schaum ausgebildet.

Nach einer weiteren bevorzugten Ausführungsform der Erfindung ist die Steuer- und Regeleinheit mit einer Pumpe oder einem Ventil verbunden, das in einer Zuflussleitung zwischen dem flexiblen Behälter und einem Vorratsbehälter mit einem Antischaummittel angeordnet ist.

Entsprechend einer weiteren bevorzugten Ausführungsform der Erfindung ist der Behälter mit Sensor als Einwegbehälter ausgebildet. Dabei ist der Behälter mit Sensor zusammenfaltbar ausgebildet und weist die oben bereits beschriebenen Vorteile auf.

Gemäß einer weiteren bevorzugten Ausführungsform der Erfindung wird der Behälter als Bioreaktor verwendet.

Weitere Merkmale und Vorteile der Erfindung ergeben sich aus der nachfolgenden speziellen Beschreibung und den Zeichnungen.

### Kurzbeschreibung der Zeichnungen

Es zeigen:

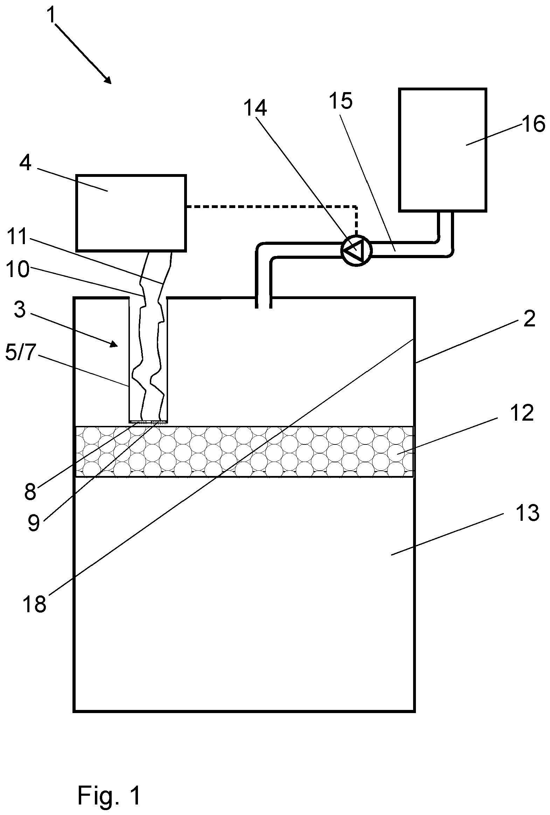

- Figur 1:: eine schematische, teilweise geschnittene Seitenansicht eines Bioreaktors mit einem Sensor, der zwei elektrisch leitende Plättchen aufweist und einer außerhalb angeordnete Steuer- und Regelungseinheit sowie einer Zuflussleitung zwischen einem Behälter mit einem Antischaummittel und dem Behälter des Bioreaktors sowie einer in der Zuflussleitung angeordneten Pumpe;

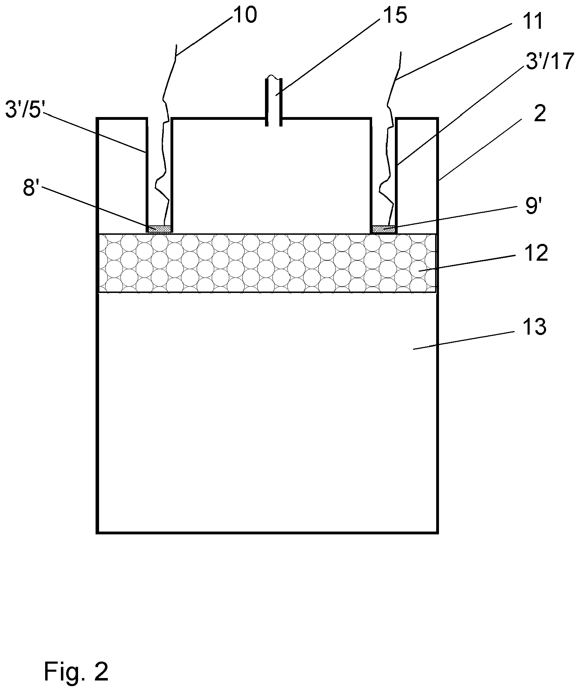

- Figur 2:: eine Seitenansicht eines weiteren Behälters mit flexibler Wandung mit einem Sensor, der zwei zylindrische Umhüllungen mit jeweils einem elektrisch leitenden Plättchen aufweist;

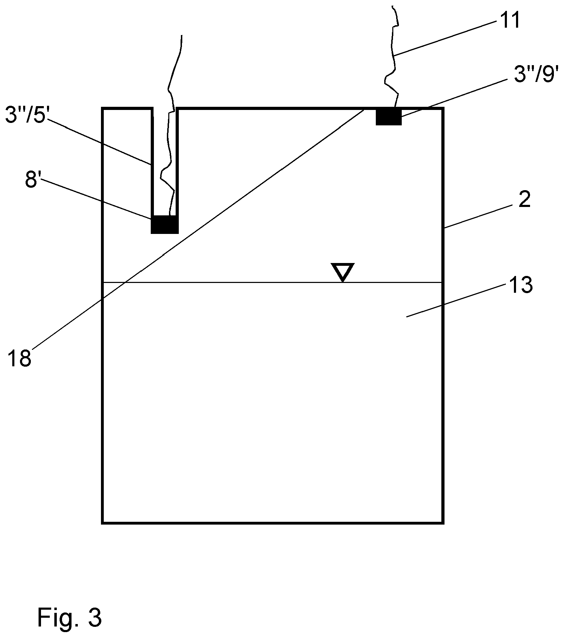

- Figur 3:: eine Seitenansicht teilweise im Schnitt eines weiteren Behälters mit flexibler Wandung, der einen Sensor aufweist, der eine zylindrische Umhüllung mit einem ersten Plättchen und ein zweites an der flexiblen Wandung des Behälters angeordnetes Plättchen aufweist;

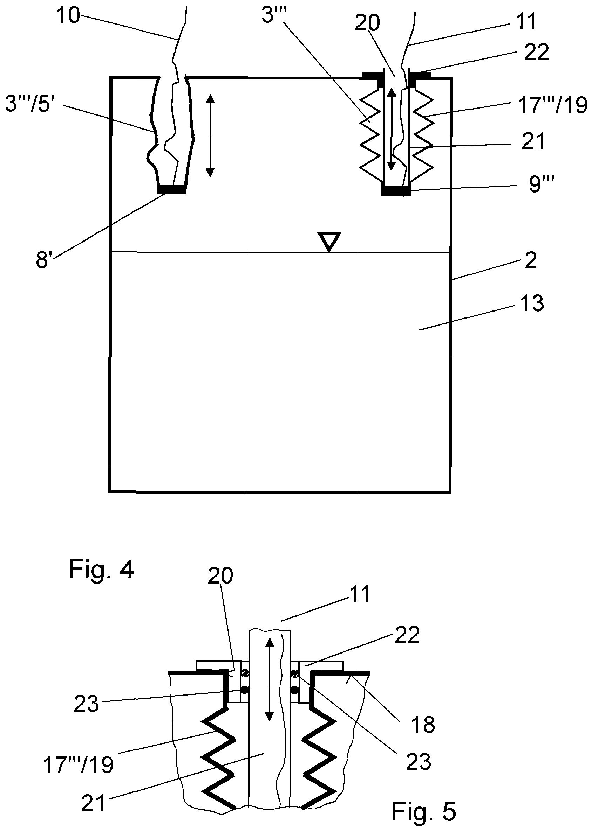

- Figur 4:: eine Seitenansicht im Schnitt eines weiteren Behälters mit flexibler Wandung, der einen Sensor aufweist, der eine flexible Umhüllung mit einem ersten Plättchen und eine zweite zylindrische Umhüllung aufweist, die als ein Faltenbalg ausgebildet ist, in der ein längsverschiebliches Rohr angeordnet ist und die an ihrem freien Ende ein zweites Plättchen aufweist;
- Figur 5:: eine Seitenansicht im Schnitt und Ausriss der zweiten zylindrischen Umhüllung von Figur 4 mit Adapterstück, Faltenbalg und verschieblichen Rohr; und

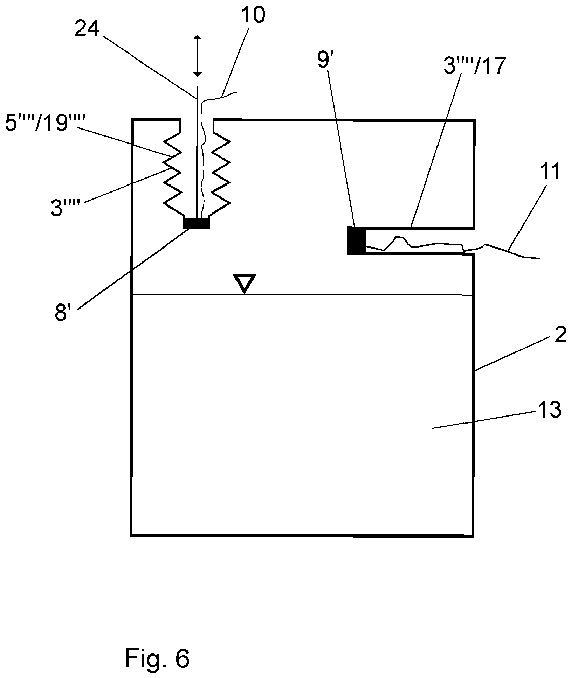

- Figur 6:: eine Seitenansicht im Schnitt eines weiteren Behälters mit flexibler Wandung mit einem Sensor, der eine erste zylindrische Umhüllung aufweist, die als ein Faltenbalg ausgebildet ist, mit einem ersten Plättchen am freien Ende des Faltenbalgs und einer zweiten zylindrischen Umhüllung mit einem zweiten Plättchen, wobei die zweite zylindrische Umhüllung quer zur ersten zylindrischen Umhüllung angeordnet ist.

### Beschreibung bevorzugter Ausführungsformen

Ein Behälter 2 mit flexibler Wandung, beispielsweise eines Bioreaktors 1, besteht im Wesentlichen aus einem Behälterinnenraum 6, einem Sensor 3 und einer Steuer- und Regelungseinheit 4.

Entsprechend dem Ausführungsbeispiel von Figur 1 besteht der Sensor 3 aus einer ersten zylindrischen Umhüllung 5, die in den Behälterinnenraum 6 hineinragt und als ein flexibler Schlauch 7 ausgebildet ist. Am freien Ende der ersten Umhüllung 5, die zylindrisch ausgebildet ist, sind entsprechend dem Ausführungsbeispiel von Figur 1 ein erstes elektrisch leitendes Plättchen 8 und beabstandet ein zweites elektrisch leitendes Plättchen 9 angeordnet. Die beiden Plättchen 8, 9 sind über Verbindungsleitungen 10, 11 mit der Steuer- und Regelungseinheit 4 verbunden. Durch Beaufschlagung mit Spannung wird die Steuer- und Regelungseinheit 4 zwischen den beiden Plättchen 8, 9 die Leitfähigkeit oder Impedanz eines die Plättchen kontaktierenden Mediums gemessen werden. Insbesondere kann dabei ermittelt werden, ob die beiden Plättchen von Gas, Schaum oder Flüssigkeit kontaktiert werden. Gegebenenfalls kann über die Steuer- und Regelungseinheit 4 eine Pumpe oder Ventil 14, das in einer Zuflussleitung 15 angeordnet ist, angesteuert werden. Die Zuflussleitung 15 verbindet dabei einen Vorratsbehälter 16, der mit einem Antischaummittel gefüllt ist, mit dem Behälter 2.

Entsprechend dem Ausführungsbeispiel der Figur 2 besteht der Sensor 3' aus einer ersten zylindrischen Umhüllung 5', die an ihrem freien Ende nur ein erstes Plättchen 8' aufweist und aus einer zweiten zylindrischen Umhüllung 17, die an ihrem freien Ende ein zweites Plättchen 9' aufweist.

Entsprechend dem Ausführungsbeispiel der Figur 3 besteht der Sensor 3" aus der ersten zylindrischen Umhüllung 5' mit dem ersten Plättchen 8' und aus dem zweiten Plättchen 9', das direkt an der flexiblen Wandung 18 des Behälters 2 angeordnet ist.

Entsprechend dem Ausführungsbeispiel der Figur 4 wird ein Sensor 3'" mit einer ersten Umhüllung 5' mit einem ersten Plättchen 8' und einer zweiten Umhüllung 17"' verwendet, die als ein Faltenbalg 19 ausgebildet ist, an dessen Ende ein zweites Plättchen 9'" angeordnet ist. Dabei ist in der als Faltenbalg 19 ausgebildeten zylindrischen Umhüllung 17"' ein gegenüber einem Wandungsdurchbruch 20 längsverschiebliches Rohr 21 angeordnet das an seinem dem Plättchen 9"' zugewandten Ende mit dem freien Ende des Faltenbalgs 19 und/oder dem Plättchen 9"' verbunden ist. Der Wandungsdurchbruch 20 weist dabei ein Adapterstück 22 auf, in dem das längsverschiebliche Rohr 21 angeordnet ist. Das längsverschiebliche Rohr 21 kann dabei über Dichtringe 23 in dem Adapterstück 22 geführt werden. Figur 5 zeigt eine vergrößerte Darstellung im Bereich des Adapterstückes 22 von Figur 4 im Ausriss.

Das Ausführungsbeispiel von Figur 6 zeigt einen Sensor 3"" mit einer ersten Umhüllung 5"", die als ein Faltenbalg 19"" ausgebildet ist und ein erstes Plättchen 8"" aufweist, das mit einer Positionierungsstange 24 verbunden ist. Über die Positionierungsstange 24 ist das erste Plättchen 8"" auf die gewünschte Messposition oder Füllstandshöhe einstellbar. Weiterhin weist der Sensor 3"" eine zweite Umhüllung 17 auf, die quer zur ersten Umhüllung 5'" angeordnet ist.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben. Insbesondere lassen sich die einzelnen Sensoren, die Anzahl der Plättchen und ihre Anordnung und Kombination variieren.

### Bezugszeichenliste

- 1: Bioreaktor
- 2: Behälter
- 3, 3', 3", 3'", 3"": Sensor
- 4: Steuer- und Regeleinheit
- 5, 5', 5"": erste Umhüllung
- 6: Behälterinnenraum von 2
- 7: flexibler Schlauch von 5
- 8, 8': erstes Plättchen
- 9, 9', 9'": zweites Plättchen
- 10: Verbindungsleitung von 8
- 11: Verbindungsleitung von 9
- 12: Schaum
- 13: Flüssigkeit
- 14: Ventil
- 15: Zuflussleitung
- 16: Vorratsbehälter
- 17, 17"': zweite Umhüllung von 3
- 18: Wandung von 2
- 19, 19"": Faltenbalg von
- 20: Wandungsdurchbruch
- 21: Rohr
- 22: Adapterstück
- 23: Dichtring
- 24: Positionierungsstange

## Patentansprüche

1. Behälter (2) mit flexibler Wandung und mindestens einem in den von der flexiblen Wandung umgebenen Behälterinnenraum (6) hineinragenden elektrischen Sensor (3, 3', 3", 3"', 3""), bei dem zwischen mindestens zwei elektrisch leitenden Plättchen (8, 8', 9, 9', 9"') die Leitfähigkeit oder Impedanz eines die Plättchen (8, 8', 9, 9', 9"') umgebenden Mediums ermittelbar ist, wobei die Plättchen (8, 8', 9, 9', 9"') über Verbindungsleitungen (10, 11) mit einer außerhalb des Behälterinnenraumes (6) angeordneten Steuer- und Regeleinheit (4) verbunden sind,
**dadurch gekennzeichnet,**
**dass** mindestens ein erstes Plättchen (8, 8') an einem geschlossenen, freien Ende einer ersten Umhüllung (5, 5', 5"") mit einer zum umgebenden Medium freiliegenden Kontaktfläche angeordnet ist und
**dass** die Umhüllung (5, 5', 5"", 17, 17'") im Behälterinnenraum in ihrer Länge verstellbar und als ein flexibler Schlauch (7) oder Faltenbalg (19, 19"") ausgebildet ist, durch den die elektrische Verbindungsleitung (10, 11) des Plättchens (8, 8', 9, 9', 9"') nach außen geführt wird.

2. Behälter nach Anspruch 1,
**dadurch gekennzeichnet,**
**dass** in der als Faltenbalg (19, 19"") ausgebildeten Umhüllung (5"",17"') ein gegenüber einem Wandungsdurchbruch (20) längs verschiebliches Rohr (21) oder eine Positionierungsstange (24) angeordnet ist, das oder die an seinem dem Plättchen (8', 9"') zugewandten Ende mit dem freien Ende des Faltenbalgs (19, 19"") verbunden ist.

3. Behälter nach Anspruch 2,
**dadurch gekennzeichnet,**
**dass** das längs verschiebliche Rohr (21) an seinem dem Plättchen (9'") abgewandten Ende aus dem Behälterinnenraum (6) herausragt.

4. Behälter nach Anspruch 2 oder 3,
**dadurch gekennzeichnet,**
**dass** der Wandungsdurchbruch (20) ein Adapterstück (22) aufweist, in dem das längs verschiebliche Rohr (21) angeordnet ist und dessen dem Behälterinnenraum (6) zugewandtes Ende mit dem Faltenbalg (19) verbunden ist.

5. Behälter nach einem der Ansprüche 1 bis 4,
**dadurch gekennzeichnet,**
**dass** das freie Ende der ersten Umhüllung (5) ein zweites Plättchen (9) aufweist, das zum ersten Plättchen (8) beabstandet angeordnet ist.

6. Behälter nach einem der Ansprüche 1 bis 4,
**dadurch gekennzeichnet,**
**dass** ein zweites Plättchen (9', 9"') an dem geschlossenen, freien Ende einer zweiten Umhüllung (17, 17"')mit einer zum umgebenden Medium freiliegenden Kontaktfläche angeordnet ist.

7. Behälter nach einem der Ansprüche 1 bis 4,
**dadurch gekennzeichnet,**
**dass** ein zweites Plättchen (9') direkt an der dem Behälterinnenraum (6) zugewandten Innenseite der flexiblen Wandung (18) angeordnet ist und eine von der Wandung (18) abgewandte freiliegende Kontaktfläche aufweist.

8. Behälter nach einem der Ansprüche 1 bis 7,
**dadurch gekennzeichnet,**
**dass** die Kontaktflächen der Plättchen (8, 8', 9, 9', 9'") oberhalb eines vorgesehenen Flüssigkeitspegels angeordnet sind.

9. Behälter nach einem der Ansprüche 1 bis 8,
**dadurch gekennzeichnet,**
**dass** der von mindestens zwei Plättchen (8, 8', 9, 9', 9"') gebildete Sensor (3, 3', 3", 3"', 3"") zur Detektion von Flüssigkeit (13) und/oder Schaum (12) ausgebildet ist.

10. Behälter nach Anspruch 9,
**dadurch gekennzeichnet,**
**dass** die Steuer- und Regeleinheit (4) mit einer Pumpe oder einem Ventil (14) verbunden ist, das in einer Zuflussleitung (15) zwischen dem flexiblen Behälter (2) und einem Vorratsbehälter (16) mit einem Antischaummittel angeordnet ist.

11. Behälter nach einem der Ansprüche 1 bis 10,
**dadurch gekennzeichnet,**
**dass** der Behälter (2) mit Sensor (3, 3', 3", 3"', 3"") als Einwegbehälter ausgebildet ist.

12. Behälter nach einem der Ansprüche 1 bis 11,
**dadurch gekennzeichnet,**
**dass** der Behälter (2) mit Sensor (3, 3', 3", 3"', 3"") zusammenfaltbar ausgebildet ist.

13. Behälter nach einem der Ansprüche 1 bis 12,
**dadurch gekennzeichnet,**
**dass** der Behälter (2) als Bioreaktor (1) verwendet wird.

## Claims

1. A container (2) with flexible walls and at least one electrical sensor (3, 3', 3", 3"', 3"") which projects into the container interior (6) which is surrounded by the flexible walls and which allows to determine the conductivity or impedance of a medium surrounding the plates (8, 8', 9, 9', 9"') between at least two electrically conductive plates (8, 8', 9, 9', 9"'), wherein the plates (8, 8', 9, 9', 9"') are connected via connecting lines (10, 11) to a control and regulating unit (4), which is arranged outside the container interior (6),
**characterized in that**
at least one first plate (8, 8') is arranged on a closed, free end of a first sheathing (5, 5', 5"") with a contact area which is exposed to the surrounding medium, and
the sheathing (5, 5', 5"", 17, 17'") is adjustable in length in the container interior and is in the form of a flexible hose (7) or folding bellows (19, 19"") through which the electrical connecting line (10,11) of the plate (8, 8', 9, 9', 9"') is routed to the outside.

2. The container according to Claim 1,
**characterized in that**
a tube (21) that is longitudinally displaceable with respect to a wall aperture (20), or a positioning rod (24), is arranged within the sheathing (5"", 17"'), which is designed as a bellows (19,19""), the end of which tube or rod facing toward the plate (8', 9"') being connected to the free end of the bellows (19, 19"").

3. The container according to Claim 2,
**characterized in that**
the end of the longitudinally displaceable tube (21) that faces away from the plate (9"') projects outside of the container interior (6).

4. The container according to Claim 2 or 3,
**characterized in that**
the wall aperture (20) has an adapter piece (22) in which the longitudinally displaceable tube (21) is arranged, the end of which tube facing the container interior (6) being connected to the bellows (19).

5. The container according to any of Claims 1 to 4,
**characterized in that**
the free end of the first sheathing (5) has a second plate (9) arranged at a distance from the first plate (8).

6. The container according to any of Claims 1 to 4,
**characterized in that**
a second plate (9', 9"') is arranged on the closed, free end of a second sheathing (17, 17"') with a contact surface which is exposed to the surrounding medium.

7. The container according to any of Claims 1 to 4,
**characterized in that**
a second plate (9') is arranged directly on the inner side of the flexible wall (18) facing the container interior (6) and has an exposed contact surface facing away from the wall (18).

8. The container according to any of Claims 1 to 7,
**characterized in that**
the contact surfaces of the plates (8, 8', 9, 9', 9"') are arranged above an intended fluid level.

9. The container according to any of Claims 1 to 8,
**characterized in that**
the sensor (3, 3', 3", 3"', 3"") formed by at least two plates (8, 8', 9, 9', 9"') is designed for the detection of fluid (13) and/or foam (12).

10. The container according to Claim 9,
**characterized in that**
the control and regulating unit (4) is connected to a pump or a valve (14) arranged in an inlet line (15) between the flexible container (2) and a reservoir (16) containing an antifoam agent.

11. The container according to any of Claims 1 to 10,
**characterized in that**
the container (2) with sensor (3, 3', 3", 3"', 3"") is designed as a single-use container.

12. The container according to any of Claims 1 to 11,
**characterized in that**
the container (2) with sensor (3, 3', 3", 3"', 3"") is designed so as to be capable of being folded up.

13. The container according to any of Claims 1 to 12,
**characterized in that**
the container (2) is used as a bioreactor (1).

## Revendications

1. Réservoir (2) comprenant une paroi flexible et au moins un capteur électrique (3, 3', 3", 3"', 3"") pénétrant dans l'espace intérieur (6) du réservoir entouré par la paroi flexible, selon lequel on peut déterminer, entre au moins deux plaquettes (8, 8', 9, 9', 9"') électriquement conductrices, la conductivité ou l'impédance d'un fluide entourant les plaquettes (8, 8', 9, 9', 9"'), sachant que les plaquettes (8, 8', 9, 9', 9"') sont reliées par l'intermédiaire de lignes de raccordement (10,11) à une unité (4) de commande et de régulation disposée en dehors de l'espace intérieur (6) du réservoir,
**caractérisé en ce qu'**au moins une première plaquette (8, 8') est disposée à une extrémité libre, fermée, d'une première gaine (5, 5', 5"") avec une surface de contact exposée au milieu environnant,
et **en ce que** la gaine (5, 5', 5"", 17, 17"') peut être réglée en longueur dans l'espace intérieur du réservoir et est réalisée sous la forme d'un tuyau flexible (7) ou d'un soufflet (19, 19""), à travers lequel la ligne de raccordement électrique (10, 11) de la plaquette (8, 8', 9, 9', 9"') est dirigée vers l'extérieur.

2. Réservoir selon la revendication 1, **caractérisé en ce qu'**une tringle de positionnement (24) ou un tube (21) longitudinalement coulissant par rapport à une ouverture de paroi (20) est disposé(e) dans la gaine (5"", 17"') réalisée sous forme de soufflet (19, 19""), tringle ou tube qui est, à son extrémité tournée vers la plaquette (8', 9"'), relié(e) à l'extrémité libre du soufflet (19, 19"").

3. Réservoir selon la revendication 2, **caractérisé en ce que** le tube (21) longitudinalement coulissant dépasse de l'espace intérieur (6) du réservoir à son extrémité opposée à la plaquette (9"').

4. Réservoir selon la revendication 2 ou 3, **caractérisé en ce que** l'ouverture de paroi (20) présente un raccord adaptateur (22), dans lequel est disposé le tube (21) longitudinalement coulissant et dont l'extrémité tournée vers l'espace intérieur (6) du réservoir est reliée au soufflet (19).

5. Réservoir selon l'une des revendications 1 à 4, **caractérisé en ce que** l'extrémité libre de la première gaine (5) présente une deuxième plaquette (9), qui est disposée à distance de la première plaquette (8).

6. Réservoir selon l'une des revendications 1 à 4, **caractérisé en ce qu'**une deuxième plaquette (9', 9"') est disposée à l'extrémité libre, fermée, d'une deuxième gaine (17, 17"') avec une surface de contact exposée au milieu environnant.

7. Réservoir selon l'une des revendications 1 à 4, **caractérisé en ce qu'**une deuxième plaquette (9') est directement disposée sur le côté intérieur de la paroi flexible (18) qui est tourné vers l'espace intérieur (6) du réservoir et présente une surface de contact exposée qui est opposée à la paroi (18).

8. Réservoir selon l'une des revendications 1 à 7, **caractérisé en ce que** les surfaces de contact des plaquettes (8, 8', 9, 9', 9'") sont disposées au-dessus d'un niveau de liquide prévu.

9. Réservoir selon l'une des revendications 1 à 8, **caractérisé en ce que** le capteur (3, 3', 3", 3"', 3"") formé par au moins deux plaquettes (8, 8', 9, 9', 9"' est conçu pour la détection d'un liquide (13) et/ou d'une mousse (12).

10. Réservoir selon la revendication 9, **caractérisé en ce que** l'unité (4) de commande et de régulation est reliée à une pompe ou une soupape (14) qui est disposée dans une conduite de flux entrant (15) entre le réservoir flexible (2) et un réservoir (16) contenant un produit antimousse.

11. Réservoir selon l'une des revendications 1 à 10, **caractérisé en ce que** le réservoir (2) pourvu du capteur (3, 3', 3", 3"', 3"") est conçu comme récipient à usage unique.

12. Réservoir selon l'une des revendications 1 à 11, **caractérisé en ce que** le réservoir (2) pourvu du capteur (3, 3', 3", 3'", 3"") est conçu repliable.

13. Réservoir selon l'une des revendications 1 à 12, **caractérisé en ce que** le réservoir (2) est utilisé comme bioréacteur (1).
